# EUROPEAN PATENT APPLICATION

(11) **EP 4 545 626 A1**
(43) Date of publication of application: **30.04.2025**
(21) Application number: 22947912.6
(22) Date of filing: 21.06.2022
(51) Int. Cl.: C12M 1/26, C12M 1/00

(54) **MICROORGANISM SEPARATION/COLLECTION DEVICE**

(71) Applicant: The Chugoku Electric Power Co., Inc., Hiroshima-shi, Hiroshima 730-8701 (JP)
(72) Inventor: NISHIDA Yurika, Hiroshima-shi, Hiroshima 730-8701 (JP); YANAGAWA Toshiharu, Hiroshima-shi, Hiroshima 730-8701 (JP); HAYASHI Yoshio, Himeji-Shi, Hyogo 672-8023 (JP); KAMIYA Kyoko, Himeji-Shi, Hyogo 672-8023 (JP); YAMASHITA Keiji, Himeji-Shi, Hyogo 672-8023 (JP)
(74) Representative: HGF
(86) International application number: PCT/JP2022/024743
(87) International publication number: WO 2023/248350

(57) **Abstract**

Provided is a microorganism separation/collection device that can easily separate/collect barnacle larvae from a sample containing several types of microorganisms. The microorganism separation/collection device for separating/collecting barnacle larvae from a sample containing several types of microorganisms comprises: a body unit formed by a light blocking member and having a space configured to store a liquid therein; a collection unit disposed above the body unit and formed by a light transmissive member; a flow passage for connecting the body unit and the collection unit; and an irradiation unit for irradiating the collection unit with visible light.

## Description

### TECHNICAL FIELD

The present invention relates to a microorganism separation and collection device.

### BACKGROUND ART

In a thermal power plant, a nuclear power plant, or the like that uses seawater as cooling water, there are cases where marine organisms such as barnacles attach to the inside of a water intake path that takes in seawater from the sea and supplies the seawater to a condenser, or a water discharge path that discharges the seawater that has passed through the condenser to the sea. When the number of marine organisms attached increases, this may cause problems such as blocking the flow path of the cooling water and reducing cooling performance. Therefore, to suppress the attachment of marine organisms, a chlorine-based chemical is conventionally injected into the cooling water.

However, adding an excessive amount of a chlorine-based chemical to the cooling water cannot be said to be preferable from the viewpoint of environmental influence and the viewpoint of cost for adding the chemical, and it is required to add an appropriate amount of a chlorine-based chemical. For example, if the number of attached organisms such as barnacles in seawater can be ascertained, it is possible to appropriately adjust the addition amount of a chlorine-based chemical according to the number of attached organisms.

To count a specific species of microorganism in water, it has been conventionally practiced to collect a sample using a plankton net in a sea area and manually perform counting using a microscope or the like. However, since the sample contains a plurality of species of phytoplankton and zooplankton, this is very time-consuming and laborious. As a method of isolating a specific species of microorganism from a sample containing a plurality of species of microorganisms, there is disclosed a method of separating and collecting microorganisms in blocks arranged in a direction in which an external stimulus is applied by applying a directional external stimulus to the microorganisms contained in an isolation container for a predetermined period of time (for example, see Patent Document 1).

### Citation List

### Patent Document

Patent Document 1: Japanese Unexamined Patent Application, Publication No. 2013-255458

### DISCLOSURE OF THE INVENTION

### Problems to be Solved by the Invention

The technique disclosed in Patent Document 1 uses, for example, a light source as an external stimulating means, but it is unclear what kind of light source should be used to isolate a desired specific species of microorganism. In a case where a specific species of microorganism is to be isolated simply by utilizing differences in phototaxis to a predetermined light source, the irradiation time also needs to be adjusted as appropriate. When the irradiation time is too long or too short, there is a possibility that the isolation cannot be performed as intended. Furthermore, no specific means for collecting the isolated sample is disclosed.

In response to the above issues, an object of the present invention is to provide a microorganism separation and collection device capable of easily separating and collecting barnacle larvae from a sample containing a plurality of species of microorganisms.

### Means for Solving the Problems

(1) The present invention relates to a microorganism separation and collection device for separating and collecting barnacle larvae from a sample containing a plurality of species of microorganisms. The device includes a body including a light shielding member and having a space capable of storing a liquid therein, a collection part that is disposed above the body and includes a light transmissive member, a flow path connecting the body and the collection part, and an irradiation unit configured to irradiate the collection part with visible light.
(2) In the microorganism separation and collection device according to (1), the body includes a first opening that is connected to the flow path and opens upward. The flow path is configured to be shieldable from external light, and has at least a part including a narrow part narrower than an inner diameter of the first opening.
(3) In the microorganism separation and collection device according to (2), the body includes a branch part that branches upward in two directions. At one branch destination of the branch part, the first opening is formed, and at the other branch destination of the branch part, a second opening is formed at a position higher than the first opening.
(4) In the microorganism separation and collection device according to (3), the first opening is closed by a first closing member having a hole through which the flow path can be inserted. The second opening is closed by a second closing member so as to be openable and closable.

### Effects of the Invention

The present invention can provide a microorganism separation and collection device capable of easily separating and collecting barnacle larvae from a sample containing a plurality of species of microorganisms.

### BRIEF DESCRIPTION OF THE DRAWINGS

FIG. 1 is a schematic cross-sectional view showing the configuration of a microorganism separation and collection device according to the present embodiment;
FIG. 2 is a schematic cross-sectional view showing the configuration of a body of the microorganism separation and collection device according to the present embodiment;
FIG. 3 is a schematic cross-sectional view showing a state at the time of separation and collection of microorganisms in the microorganism separation and collection device according to the present embodiment; and
FIG. 4 is a schematic cross-sectional view showing the configuration of a microorganism separation and collection device according to another embodiment.

### PREFERRED MODE FOR CARRYING OUT THE INVENTION

### <<First Embodiment>>

### <Microorganism Separation and Collection Device>

A microorganism separation and collection device 1 according to the present embodiment can selectively and preferably collect barnacle larvae from a sample containing a plurality of species of microorganisms collected from a water body such as the ocean.

Barnacles is a generic term for organisms classified into Arthropoda, Crustacea, Cirripedia, and Thoracica, and includes, for example, organisms belonging to Balanomorpha, which includes Amphibalanus amphitrite, Amphibalanus eburneus, Megabalanus rosa, Balanus trigonus, Megabalanus volcano, Amphibalanus reticulatus, Chthamalus challengeri, Balanus albicostatus, and Amphibalanus improvisus.

Barnacles swim in the sea during their larval stage, and the initial larvae are referred to as nauplius larvae and the growing larvae are referred to as cypris larvae. Cypris larvae are equivalent to attachment-stage larvae, and attach to a suitable substrate after swimming and metamorphose into adults. Cypris larvae attach to underwater structures, such as seawater intake or discharge facilities at power plants, coastal aquaculture facilities, and fishing facilities, and then grow thereon, potentially causing adverse effects on these facilities. Therefore, by ascertaining the number of cypris and nauplius larvae of barnacles living in the sea area where these facilities exist, it is possible to take necessary measures, such as injecting chlorine-based chemicals.

The present inventors' studies have revealed that cypris larvae and nauplius larvae exhibit strong positive phototaxis to visible light. The microorganism separation and collection device according to the present embodiment can easily separate and collect cypris larvae and nauplius larvae of barnacles by utilizing the strong positive phototaxis of the larvae.

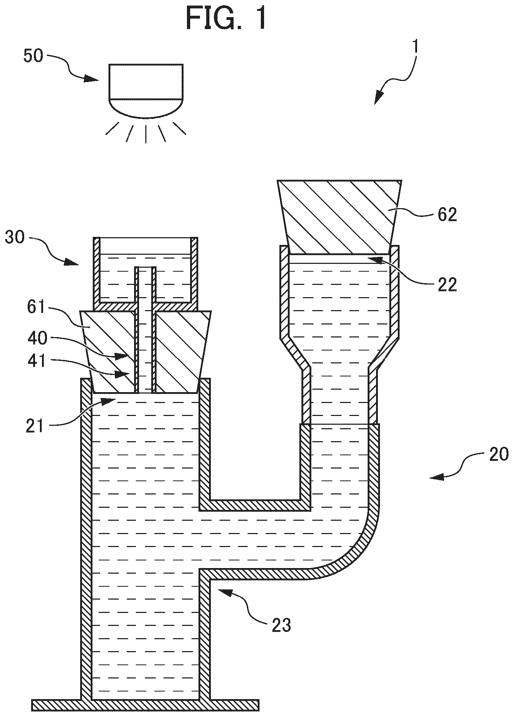

As shown in FIG. 1, the microorganism separation and collection device 1 includes a body 20, a collection part 30, a flow path 40, and an irradiation unit 50.

The body 20 is a hollow member having a space capable of storing a liquid (a sample such as seawater containing a plurality of species of microorganisms) therein. As shown in FIG. 1, the body 20 has a shape in which the lower side of the internal space is closed in a state of being installed on the installation surface, and the body branches upward in two directions at a branch part 23. A first opening 21 and a second opening 22 are respectively formed at the branch destinations of the branch part 23. The first opening 21 is closed by a first closing member 61 so as to be openable and closable, the first closing member 61 having a hole through which the flow path 40 can be inserted. The first opening 21 is also connected to the flow path 40, so that the body 20 and the collection part 30 are in communication with each other. The first opening 21 is preferably openable and closable, but does not have to be openable and closable. The second opening 22 is closed by the second closing member 62 so as to be openable and closable. The upper end of the second opening 22 is disposed at a position higher than the first opening 21 and higher than the upper end of the flow path 40 in the installed state. The member in which the second opening 22 is formed may be configured as a separate body from the body 20, or may be formed integrally with the body 20.

The body 20 includes a light shielding member through which light cannot pass. The light shielding member is not limited as long as it is a material that does not transmit light, and a resin such as vinyl chloride, a metal, or the like can be used.

The collection part 30 is a substantially cylindrical member installed above the body 20, and the bottom surface thereof is connected to the flow path 40 in the installed state. The method of connecting the collection part 30 and the flow path 40 is not limited. For example, a method of inserting the flow path 40 into a hole formed in the bottom of the collection part 30 and sealing the periphery of the hole with a sealing material or the like so that water cannot enter and exit is exemplified. The collection part 30 can include, for example, a light transmissive member for observing microorganisms inside the collection part 30. The light transmissive member is not limited, and examples thereof include a light transmissive resin such as an acrylic resin and glass. The collection part 30 may not be a light transmissive member. In the present embodiment, the upper surface of the collection part 30 is open. On the other hand, an openable and closable lid including the light transmissive member may be provided on the upper surface of the collection part 30.

The flow path 40 connects the first opening 21 of the body 20 and the bottom surface of the collection part 30. The flow path 40 is, for example, a tubular body including a light shielding member through which light cannot pass. The flow path 40 is inserted into the hole formed in the first closing member 61. A part of the flow path 40 extends to the inside of the collection part 30, and the upper end of the flow path 40 opens in the inside of the collection part 30.

It is preferable that a narrow part 41 narrower than the inner diameter of the first opening 21 is formed in at least a part of the flow path 40. This allows only the cypris larvae that have phototaxis to the light irradiated from the irradiation unit 50 to move to the collection part 30. In addition, it is possible to easily collect the sample containing the cypris larvae that have moved to the collection part 30. In the present embodiment, the flow path 40 is a tubular body having a diameter smaller than the inner diameter of the first opening 21, and the entire flow path 40 forms the narrow part 41. On the other hand, one or more narrow parts 41 may be formed in a part of the flow path 40.

The irradiation unit 50 irradiates the collection part 30 with visible light. This makes it possible to attract cypris larvae and nauplius larvae of barnacles, which have positive phototaxis to the light, to the collection part 30. On the other hand, since other zooplankton, phytoplankton, and other matter, which do not have positive phototaxis to the above visible light, remain inside the body 20, a sample excluding these can be collected. The specific configuration of the irradiation unit 50 is not limited, and for example, an LED irradiation device, a halogen lamp, a mercury lamp, a fluorescent tube, or the like can be used, but an LED irradiation device is preferably used.

The first closing member 61 has a hole through which the flow path 40 can be inserted, and includes an elastic material such as rubber or elastomer. The first closing member 61 preferably is not light transmissive. The first closing member 61 may be formed integrally with the flow path 40. A part of the lower end of the first closing member 61 is fitted into the first opening 21 and fixed, and the upper end surface of the first closing member 61 is in contact with and fixed to the bottom surface of the collection part 30.

The second closing member 62 has the same configuration as the first closing member 61 except that it does not have a hole. A part of the lower end of the second closing member 62 is fitted into and fixed to the second opening 22.

### <Microorganism Separation and Collection Method>

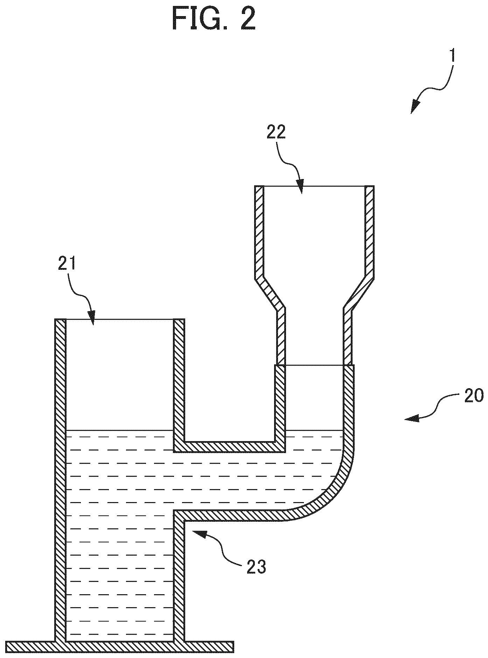

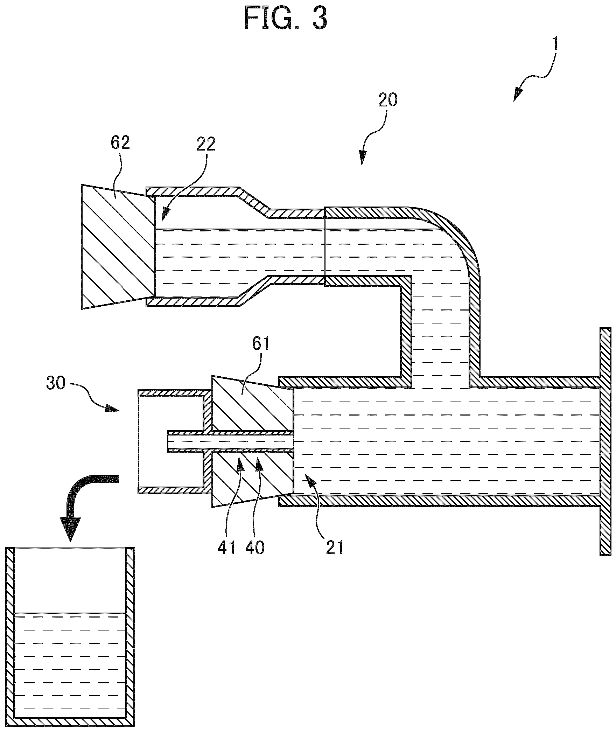

Next, a procedure for separating and collecting microorganisms using the microorganism separation and collection device 1 will be described. First, as shown in FIG. 2, the collection part 30, the flow path 40, the first closing member 61, and the second closing member 62 are removed from the body 20. In this state, a sample (plankton net sample) is introduced through the first opening 21 or the second opening 22. Note that only the second closing member 62 may be removed without removing the collection part 30, the flow path 40, and the first closing member 61, and the sample may be introduced through the second opening 22. Next, the flow path 40 and the first closing member 61 are fitted into the first opening 21 to close the first opening 21, and the collection part 30 is connected thereabove. In this state, filtered seawater is poured in through the second opening 22, and the pouring of filtered seawater is continued until the water level reaches or exceeds the upper end of the flow path 40 inside the collection part 30. Thereafter, the second closing member 62 is fitted into the second opening 22 to close the second opening 22, and the collection part 30 is irradiated with visible light by the irradiation unit 50. After the irradiation is performed for a predetermined period of time (e.g., 15 minutes or longer), the microorganism separation and collection device 1 is inclined as shown in FIG. 3 to transfer the liquid in the collection part 30 into a predetermined container.

Here, the openings of the body 20 are closed by the first closing member 61 and the second closing member 62, and only the leading end of the flow path 40 communicates with the outside. In this state, when the microorganism separation and collection device 1 is inclined and the liquid in the collection part 30 is transferred into a predetermined container, the pressure on the inside of the body 20 becomes negative, and surface tension acts on the leading end of the flow path 40. Accordingly, even when the microorganism separation and collection device 1 is inclined, the liquid stored in the body 20 is not discharged to the outside, and only the liquid in the collection part 30 is discharged, so that the liquid in the collection part 30 can be easily collected.

Next, the configuration of a microorganism separation and collection device 1a according to a second embodiment of the present invention will be described. The same components as those of the microorganism separation and collection device 1 according to the first embodiment are denoted by the same reference numerals, and the descriptions thereof may be omitted.

<<Second Embodiment>>

### <Microorganism separation and collection device>

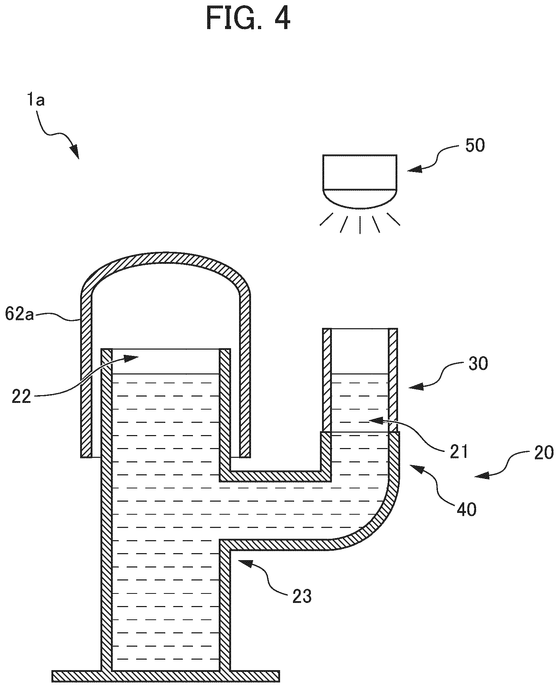

As shown in FIG. 4, the microorganism separation and collection device 1a according to the second embodiment includes a body 20, a collection part 30, a flow path 40, and an irradiation unit 50.

The body 20 is a hollow member having a shape that branches upward in two directions at a branch part 23, similarly to the microorganism separation and collection device 1. A first opening 21 and a second opening 22 are respectively formed at the branch destinations of the branch part 23. In the present embodiment, the second opening 22 is light-shielded by a light shielding member 62a so as to be openable and closable. The light shielding member 62a is not limited as long as it is capable of light-shielding the second opening 22, and for example, a cap having an inner diameter larger than the outer diameter of the second opening 22 may be disposed so as to cover the second opening 22.

In the present embodiment, the flow path 40 is a flow path of the body 20 that branches from the branch part 23. The first opening 21 is formed at an upper end of the flow path 40. The first opening is connected to the lower surface of the collection part 30. In the present embodiment, the collection part 30 is a cylindrical body including a light transmissive member, and the lower surface thereof is open.

### <Microorganism Separation and Collection Method>

As a procedure for separating and collecting microorganisms using the microorganism separation and collection device 1a, as in the first embodiment, a sample (plankton net sample) and, as necessary, filtered seawater are introduced into the body 20 until the water surface rises to the position of the collection part 30, and the second opening 22 is covered with the light shielding member 62a. Next, the collection part 30 is irradiated with visible light by the irradiation unit 50. After the irradiation is performed for a predetermined period of time (e.g., 15 minutes or longer), the liquid in the collection part 30 is collected with a dropper or the like.

Preferred embodiments of the present invention have been described above. The present invention is not limited to the above-described embodiments, and modifications, improvements, and the like within a range in which the object of the present invention can be achieved are included in the present invention.

### EXAMPLES

Hereinafter, the present invention will be described in detail with reference to Examples. However, the present invention is not limited to these Examples.

### [Collection Test of Cypris Larvae]

Using the microorganism separation and collection device 1a according to the second embodiment, a collection test of cypris larvae of Amphibalanus amphitrite was performed. The cypris larvae of Amphibalanus amphitrite obtained by breeding were introduced into the microorganism separation and collection device 1a together with seawater, and the collection part 30 was irradiated with visible light from the irradiation unit 50. A halogen lamp (peak wavelength: 646 nm, wavelength range: 360 to 760 nm, manufactured by PHILIPS 15V 150W Model No. 6423) was used as a light source. The number of cypris larvae that appeared in the collection part 30 was counted with the time when the second opening 22 was covered with the light shielding member 62a as the start time. The test was performed twice, and in the first test, 16 cypris larvae were confirmed in the collection part 30 at the end (after 17 minutes and 20 seconds had elapsed). In the second test, 13 cypris larvae were confirmed in the collection part 30 at the end (after 15 minutes and 26 seconds had elapsed).

### [Collection Rate of Cypris Larvae]

A plankton sample was obtained using a Kitahara's surface plankton net (30 cm diameter, 0.1 mm mesh; RIGOSHA 5511) (seawater filtration volume 200 L). Several tens of cypris larvae and nauplius larvae of Amphibalanus amphitrite obtained by breeding were added to this to prepare a test sample. This was used to separate a collected fraction that was collected from the collection part 30 after 15 minutes using the same procedure as in the collection test of cypris larvae, and a fraction containing other matter that remained in the body 20 (non-collected fraction). About 15 mL of the collected fraction was collected from the collection part 30 using a pipette. The fraction containing other matter was received and collected using a 60 µm mesh. The numbers (n) of cypris larvae and other planktons in the respective fractions were counted using plankton counting plates (RIGOSHA) under a stereomicroscope (OLYMPUS SZX7). The test was conducted twice. The results are shown in Table 1.

**[Table 1]**

| Type of Microorganism | First Test | | | Second Test | | | Average |
|---|---|---|---|---|---|---|---|
| | Collected Fraction (n) | Fraction Containing Other Matter (n) | Collection Rate (%) | Collected Fraction (n) | Fraction Containing Other Matter (n) | Collection Rate (%) | Collection Rate (%) |
| Cypris larva | 22.0 | 32.0 | 40.7 | 13.0 | 16.0 | 44.8 | 42.8 |
| Nauplius larva | 495.0 | 580.0 | 46.0 | 249.0 | 656.0 | 27.5 | 36.8 |
| Copepoda | 80.0 | 624.0 | 11.4 | 28.0 | 556.0 | 4.8 | 8.1 |
| Other crustaceans | - | 4.0 | 0.0 | 1.0 | 4.0 | 20.0 | 10.0 |
| Lugworms (nechtochaeta larva) | 5.0 | 84.0 | 5.6 | 13.0 | 96.0 | 11.9 | 8.8 |
| Larvacea | 1.0 | 76.0 | 1.3 | - | 30.0 | 0.0 | 0.6 |
| Arrowworm | - | 64.0 | 0.0 | - | 68.0 | 0.0 | 0.0 |
| Bivalve | - | 4.0 | 0.0 | - | 4.0 | 0.0 | 0.0 |
| Snail | - | 16.0 | 0.0 | - | - | - | 0.0 |
| Noctiluca | 1.0 | - | 100.0 | - | - | - | 50.0 |
| Others (unknown) | - | 8.0 | 0.0 | - | - | - | 0.0 |

As shown in Table 1, by using the microorganism separation and collection device according to the present embodiment, it was possible to obtain collection rates of cypris larvae and nauplius larvae of 30 to 40%. The collection rates of other crustaceans such as Copepoda were low, even though they were the same crustaceans. The collection rates of non-crustaceans such as lugworms and Larvacea were even lower. From the above results, it is clear that the microorganism separation and collection device according to the present embodiment can be used to separate cypris larvae and nauplius larvae of barnacles from other microorganisms.

### EXPLANATION OF REFERENCE NUMERALS

1, 1a microorganism separation and collection device
20 body
21 first opening
22 second opening
23 branch part
30 collection part
40 flow path
41 narrow part
50 irradiation unit
61 first closing member
62 second closing member

## Claims

1. A microorganism separation and collection device for separating and collecting barnacle larvae from a sample containing a plurality of species of microorganisms, the device comprising:
a body comprising a light shielding member and having a space capable of storing a liquid therein;
a collection part that is disposed above the body and comprises a light transmissive member;
a flow path connecting the body and the collection part; and
an irradiation unit configured to irradiate the collection part with visible light.

2. The microorganism separation and collection device according to claim 1,
wherein the body comprises a first opening that is connected to the flow path and opens upward, and
wherein the flow path is configured to be shieldable from external light, and has at least a part comprising a narrow part narrower than an inner diameter of the first opening.

3. The microorganism separation and collection device according to claim 2,
wherein the body comprises a branch part that branches upward in two directions,
wherein, at one branch destination of the branch part, the first opening is formed, and
wherein, at the other branch destination of the branch part, a second opening is formed at a position higher than the first opening.

4. The microorganism separation and collection device according to claim 3,
wherein the first opening is closed by a first closing member having a hole through which the flow path can be inserted, and
wherein the second opening is closed by a second closing member so as to be openable and closable.
